## Europäisches Patentamt

## European Patent Office

## Office européen des brevets

(19)

(11) Veröffentlichungsnummer: **0 145 850**
**B1**

(12) # EUROPÄISCHE PATENTSCHRIFT

(45) Veröffentlichungstag der Patentschrift:
**17.11.88**

(51) Int. Cl.⁴: **C 07 C 19/045**, C 07 C 17/02, C 01 B 7/07 // C07C17/156, C07C17/10

(21) Anmeldenummer: **84110531.5**

(22) Anmeldetag: **05.09.84**

(54) Verfahren zur Aufbereitung von Chlorwasserstoff als Einsatzstoff für den Ethylenoxichlorierungsprozess.

(30) Priorität: **05.09.83 DE 3331962**

(43) Veröffentlichungstag der Anmeldung:
**26.06.85 Patentblatt 85/26**

(45) Bekanntmachung des Hinweises auf die Patenterteilung:
**17.11.88 Patentblatt 88/46**

(84) Benannte Vertragsstaaten:
**DE FR GB IT NL**

(56) Entgegenhaltungen:
**DD - A - 86 387**
**DD - A - 126 192**
**DE - A - 2 649 533**
**DE - B - 1 793 835**
**DE - B - 2 556 521**

(73) Patentinhaber: **WACKER-CHEMIE GMBH,
Prinzregentenstrasse 22, D-8000 München 22 (DE)**

(72) Erfinder: **Schmidhammer, Ludwig, Dr. Dipl.-Chem.,
Pappelweg 5, D-8261 Haiming/Marktl (DE)**
Erfinder: **Dummer, Gerhard, Dipl.-Ing., Lohnerstrasse 12,
D-8261 Burgkirchen/Alz (DE)**
Erfinder: **Strasser, Rudolf, Dr. Dipl.-Chem., Lindacher
Strasse 58, D-8263 Burghausen (DE)**
Erfinder: **Haselwarter, Klaus, Dipl.-Ing. (FH), Marktler
Strasse 7, D-8263 Burghausen (DE)**
Erfinder: **Klaus, Hermann, Dipl.-Ing. (FH),
Eichenstrasse 12, D-8261 Marktl (DE)**
Erfinder: **Pichl, Eduard, Mehring 6, D-8261 Mehring (DE)**

**Beschreibung**

Die Erfindung betrifft ein Verfahren zur Aufbereitung von bei Chlorierungsreaktionen anfallendem Chlorwasserstoff für den Ethylenoxichlorierungsprozeß durch Umsetzen von im Chlorwasserstoff enthaltenem Chlor mit Ethylen in der Gasphase in Gegenwart von Trägerkatalysatoren auf Basis Übergangsmetallchlorid, wobei Eisenchlorid ausgeschlossen ist.

Chlorwasserstoff wird zur Herstellung von Dichlorethan im Oxichlorierungsprozeß eingesetzt. Dieser Chlorwasserstoff fällt in großen Mengen, aber auch in erforderlicher Reinheit, bei der thermischen Spaltung von Dichlorethan zu Vinylchlorid an. Im Hinblick auf die modernen integrierten Produktionssysteme ist es nun wünschenswert, auch Chlorwasserstoff für Oxichlorierungsreaktionen einzusetzen, der z.B. bei Perchlorierungsreaktion anfällt. Chlorwasserstoff aus solchen Quellen enthält jedoch neben tensionsgemäß vorhandenen perchlorierten Verbindungen, wie Tetrachlorkohlenstoff und Perchlorethylen, größere Mengen – bis zu 20 Mol-% – an elementarem Chlor. Durch diese Verunreinigungen wird der Chlorwasserstoff als Einsatzstoff für eine Oxichlorierung unbrauchbar: so kommt es z.B. zu Chlorabbrand in den Zuleitungen. Der Chlorgehalt schafft ferner allenfalls mit hohem, unwirtschaftlichem Aufwand zu lösende verfahrenstechnische Probleme an der einem Oxichlorierungsreaktor vorzuschaltenden Verdichtereinheit. Darüberhinaus fördert Chlor den Kornzerfall des Oxichlorierungskatalysators mit der Folge, daß innerhalb kurzer Zeit Betriebsbedingungen in Kauf genommen werden müssen, die zu erhöhter Nebenproduktbildung beim Oxichlorierungsprozeß führen. Ein höherer Anteil an perchlorierten Verbindungen, wie beispielsweise Tetrachlorkohlenstoff, führt zu ähnlichen Schädigungen des Katalysators.

Neben einer Reihe anderer physikalisch-chemischer und chemischer Maßnahmen wurde deshalb bereits vorgeschlagen, das im Chlorwasserstoff mitgeführte Chlor mit Ethylen zu Dichlorethan umzusetzen:

Gemäß DE-AS 25 56 521 wird mit Chlor verunreinigter Chlorwasserstoff mit überschüssigem Ethylen bei erhöhten Temperaturen über Aktivkohlefilter geleitet, wobei Chlor mit Ethylen zu Dichlorethan abreagiert und andere tensionsgemäß mitgeführte Verunreinigungen adsorptiv entfernt werden. Derartige Aktivkohlefilter sind jedoch sehr störanfällig, so daß stets ein Parallelbetrieb mehrerer derartiger Filter aufrechterhalten werden muß. Berücksichtigt man, daß ständig aufwendige Maßnahmen zur Regeneration dieser Filter erforderlich sind, ist das vorgeschlagene Verfahren allenfalls dann wirtschaftlich, wenn lediglich geringe Verunreinigungen im Chlorwasserstoff zu entfernen sind.

Gemäß DE-OS 22 51 696 wird Chlor aus einem gasförmigen Stoffgemisch durch Umsetzen des Chlors mit Ethylen in Gegenwart von Lewis-Säure, insbesondere Eisen-III-Chlorid, entfernt. Nachteiligerweise bilden sich unter diesen Bedingungen unerwünschte Nebenprodukte, wie Ethylchlorid und Trichlorethan, so daß das Reaktionsgemisch vor seiner Aufarbeitung destillativ gereinigt werden muß.

Gemäß DD-PS 86 387 wird das Problem, ausreichende Selektivität der Reaktion von Chlor und Ethylen zu 1,2-Dichlorethan zu erzielen, dadurch gelöst, daß ein Trägerkatalysator auf Basis Kupfer-II-Chlorid, der zusätzlich weitere Metallchloride als Promotoren enthält, eingesetzt wird, das Reaktionsgemisch mit Inertgas verdünnt wird und weiterhin die Katalysatorschüttung mit bis zu 80 Vol.-% eines Intermaterials mit hoher Wärmeleitfähigkeit, wie z.B. Siliciumcarbid, abgemagert wird. Dadurch wird jedoch wertvoller und teurer Reaktionsraum verschenkt, was sich in niedrigen Raum-Zeit-Leistungen ausdrückt. Durch die starke Verdünnung des Katalysators mit Inertmaterial müssen zur Erzielung vollständiger Umsätze auch verhältnismäßig hohe Verweilzeiten angewendet werden, mit der Folge, daß zur Erreichung technisch interessanter Durchsätze große Katalysatorvolumina notwendig sind. Nachdem in der Praxis Temperaturen um 200 °C gebraucht werden, um den Katalysatorraum voll zu nutzen, müssen zudem unwirtschaftlich geringe Standzeiten des Katalysators in Kauf genommen werden.

Aufgabe der Erfindung war es, ein Verfahren zur Aufbereitung von mit Chlor und chlorierten Verbindungen verunreinigtem Chlorwasserstoff aufzufinden, wobei Chlor durch Umsetzung mit Ethylen zu 1,2-Dichlorethan quantitativ entfernt werden kann und ferner der Chlorwasserstoff so weit von tensionsgemäß mitgeführten chlorierten Verbindungen befreit wird, daß er als Einsatzstoff für eine Ethylenoxichlorierung verwendet werden kann. Es wurde nun gefunden, daß der Chloranteil im Chlorwasserstoff mit Ethylen praktisch quantitativ mit hoher Selektivität zu 1,2-Dichlorethan umgesetzt werden kann an einem Übergangsmetallchlorid-Trägerkatalysator mit in Strömungsrichtung ansteigendem Aktivitätsprofil, wenn relativ hohe Raumströmungsgeschwindigkeiten und geringe Kontaktzeiten eingehalten werden.

Gegenstand der Erfindung ist ein Verfahren zur Aufbereitung von bei Chlorierungsreaktionen anfallendem Chlorwasserstoff für den Ethylenoxichlorierungsprozeß durch Umsetzen von im Chlorwasserstoff enthaltenem Chlor mit Ethylen in der Gasphase in Gegenwart von Trägerkatalysatoren auf Basis Übergangsmetallchlorid, wobei Eisenchlorid ausgeschlossen ist, das dadurch gekennzeichnet ist, daß das im Chlorwasserstoff enthaltene Chlor bei Temperaturen von 70 bis 150 °C, einem Druck von 1,5 bis 5 bar abs., mit einem 5 bis 20%igen molaren Überschuß an Ethylen in Gegenwart eines Trägerkatalysators auf Basis Übergangsmetallchlorid mit in Strömungsrichtung ansteigendem Aktivitätsprofil zur Reaktion gebracht wird, wobei Raumgeschwindigkeiten von 1000 $h^{-1}$ bis 3000 $^{-1}$ und Kontaktzeiten von 2 bis 5 Sekunden eingehalten werden, und das Reaktionsprodukt nach Austritt aus dem Reaktor einer Teilkondensation unterworfen wird, wobei in zumindest einem Kondensationsschritt eine Temperatur von −20 bis −30 °C eingehalten wird.

Vorzugsweise beträgt die Reaktionstemperatur 90 bis 130 °C. Die Raumströmungsgeschwindigkeiten betragen bevorzugt 1200 $h^{-1}$ bis 1800 $h^{-1}$. Die Raumströmungsgeschwindigkeit ist definiert als die Menge, ausgedrückt in Volumen gasförmigen Reaktionsgemisches bei 0 °C und 760 Torr Druck, die pro Stunde und pro 1 Liter Katalysatorvolumen das Reaktionssystem durchströmt.

Vorzugsweise beträgt der Überschuß an Ethylen, bezogen auf die Stöchiometrie anwesenden Chlors im Chlorwasserstoff, 8 bis 12 Mol-%.

Der erfindungsgemäß aufzubereitende Chlorwasserstoff fällt als an sich unerwünschtes Nebenprodukt bei Chlorierungsreaktionen an und weist neben tensionsgemäß mitgeführten Chlorierungsprodukten einen Chloranteil von bis zu 20 Mol-% auf. Beispiele für derartige Chlorierungsprozesse sind insbesondere Perchlorierungsprozesse von $C_1$ bis $C_3$ Kohlenwasserstoffen. Weitere Beispiele sind Chlorierungen von Aromaten und von Essigsäure in Gegenwart von Acetanhydrid oder Acetylchlorid.

Erfindungsgemäß wird der Chloranteil durch Reaktion mit überschüssigem Ethylen in 1,2-Dichlorethan übergeführt. Das Ethylen wird am besten kurz vor Eintritt des ggf. in etwa auf die gewünschte Reaktionstemperatur vorgewärmten Gasgemisches in den Reaktor zugegeben.

Es wird ein Trägerkatalysator eingesetzt, der als aktive Komponente Übergangsmetallchlorid und vorzugsweise zusätzlich Promotoren aufweist. Beispiele für Übergangsmetallchloride sind Kupfer-II-chlorid, Mangan-II-chlorid, Chrom-III-chlorid, Kobalt-II-chlorid, Lanthan-III-chlorid, insbesondere Kupfer-II-chlorid. Eisen-III-chlorid wird ausdrücklich ausgeschlossen, da es unter den Bedingungen des erfindungsgemäßen Verfahrens in nicht tragbarem Maße die Nebenproduktbildung fördert.

Als Promotoren kommen beispielsweise die Chloride des Kaliums, des Magnesiums, Calciums und des Silbers in Betracht.

Als Trägermaterial werden an sich bekannte Inertmaterialien, wie Aluminiumoxid, Kieselgel, Alumosilikate und dergleichen, verwendet. Das Trägermaterial liegt vor in der Form von Kugeln, Kegeln, Würfeln, Hohlsträngen und dergleichen.

Erfindungsgemäß weist der Katalysator ein in Strömungsrichtung steigendes Aktivitätsprofil auf, das dadurch dargestellt wird, daß die Menge an Aktivsubstanz, bezogen auf das Gesamtgewicht des Katalysators in Strömungsrichtung erhöht wird. Bei gegebener Aktivsubstanzkonzentration am Reaktoreintritt von 5 bis 7 Gew.-% steigt die Aktivsubstanzkonzentration bis zum Reaktoraustritt, kontinuierlich oder diskontinuierlich (z.B. in 2 bis 4 Sektoren), auf das 2,5- bis 4fache. Die Konzentration an Promotoren beträgt zumeist einheitlich 1,5 bis 3,5 Gew.-%, bezogen auf das Gesamtgewicht des Katalysators.

Die Reaktionstemperaturen werden auf 70 bis 150 °C, insbesondere 90 bis 130 °C, eingestellt. Der Druck beträgt 1,5 bis 5 bar. Die Einhaltung der weiteren Parameter-Raumströmungsgeschwindigkeiten und Kontaktzeiten von 2 bis 5 Sekunden werden in der dem Fachmann geläufigen Weise durch die Abmessungen der Apparaturen, insbesondere durch die Wahl der Rohrlängen und -querschnitte sowie durch Bereitstellung eines entsprechenden Katalysatorvolumens gewährleistet.

Nach dem Verlassen des Reaktors wird das Reaktionsprodukt einer Teilkondensation bei Temperaturen von −20 bis −30 °C unterworfen. Hierbei wird im wesentlichen Chlorwasserstoff, Ethylen und ggf. weitere nicht kondensierbare Anteile von tensionsgemäß mitgeführten chlorierten Verbindungen und insbesondere von entstandenem 1,2-Dichlorethan getrennt. Zweckmäßigerweise wird die Teilkondensation in mindestens 2 Kondensationsstufen durchgeführt. Beispielsweise wird in einer ersten Kondensationsstufe das Reaktionsprodukt über einen mit Wasser betriebenen Wärmetauscher geleitet, wobei Temperaturen von etwa 20 bis 40 °C eingehalten werden.

Das vom Wärmetauscher abfließende Gemisch aus kondensierten und nicht kondensierten Bestandteilen kann anschließend in einem Gas/Flüssigkeitsabscheider gesammelt werden. Der nicht kondensierte Anteil strömt danach zu einem mit beispielsweise Frigen beaufschlagten zweiten Wärmetauscher, in dem bei einer Temperatur von −20 bis −30 °C eine praktisch vollständige Kondensation der kondensierbaren Bestandteile des Reaktionsgemisches stattfindet. Der nicht kondensierte Abgasstrom, im wesentlichen Chlorwasserstoff, wird anschließend, ohne daß weitere Reinigungsschritte erforderlich wären, einer Ethylenoxichlorierung zugeführt.

Das im erfindungsgemäßen Verfahren anfallende Kondensat, im wesentlichen 1,2-Dichlorethan, kann ebenfalls, ohne daß weitere Reinigungsschritte erforderlich wären, z.B. einer thermischen Spaltung zu Vinylchlorid zugeführt werden.

Die Erfindung wird nun anhand von Beispielen und Vergleichsbeispielen näher erläutert:

Beispiel 1

3884 Nm³/h Chlorwasserstoff aus einem Perchlorierungsprozeß verunreinigt mit 19 Mol-% Chlor, 0,43 Vol.-% Tetrachlorkohlenstoff und 45 Vol.-ppm Perchlorethylen wurden über eine Dampfmantelbeheizung auf 100 °C vorgewärmt und druckgeregelt bei 2,5 bar absolut unmittelbar vor Eintritt in einen Reaktor mit 810 Nm³/h Ethylen, das unter einem Druck von 3 bar absolut stand und auf einer Temperatur von 40 °C gehalten wurde, vermischt. Der Reaktor bestand aus einem Bündel von Nickelrohren (1570 Rohre mit einer lichten Weite von 27,5 mm und einer Länge von 3660 mm, entsprechend einem Katalysatorvolumen von 3200 l). In den Nickelrohren befand sich der Katalysator. Als Trägermaterial wurde Aluminiumoxid verwendet, das in Form von 3 bis 5 mm großen Kugeln vorlag. Als Aktivmaterial wurde Kupfer-II-chlorid herangezogen. Das in Strömungsrichtung ansteigende Aktivitätsprofil wurde dadurch verwirklicht, daß im Eingangsdrittel die Konzentration an Kupfer-II-chlorid 6,5 Gew.-% und im mittleren Drittel 11 Gew.-% und im letzten Drittel 19 Gew.-% betrug. Ferner war der Trägerkatalysator einheitlich mit 2 Gew.-% Kaliumchlorid imprägniert. Im Bereich des unteren und oberen Rohrbodens befanden sich weiterhin inerte Sattelkörper aus Keramik.

Die Nickelrohre waren mit einem Außenmantel umgeben, indem sich als Kühlmedium zur Abführung der freiwerdenden Reaktionswärme Wasser befand, das unter einem Dampfdruck von 2,25 bar absolut entsprechend einer Temperatur von 125 °C stand.

Die Raumströmungsgeschwindigkeit betrug unter Normalbedingungen 1698 h⁻¹, die Verweilzeit des Reaktionsgemisches im Reaktor 2,6 Sekunden.

Nach Verlassen des Reaktors strömte das Reaktionsgemisch in einen mit Wasser gekühlten Wärmetauscher, wo bei 38 °C eine Teilkondensation eintrat. Das vom Wärmetauscher abfließende Gemisch aus kondensierten und nicht kondensierten Bestandteilen wurde in einem Gas-Flüssigkeitsabscheider gesammelt. Der nicht kondensierte Anteil strömte zu einem mit Frigen beaufschlagten zweiten Wärmetauscher, wo bei minus 25 °C eine praktisch vollständige Kondensation der kondensierbaren Bestandteile des Reaktionsgemisches stattfand. Das kalte Kondensat wurde in einem zweiten Gas-Flüssigkeitsabscheider gesammelt, während der nicht kondensierte Abgasstrom, im wesentlichen Chlorwasserstoff, druckgeregelt bei 1,2 bar absolut einer Verdichterstation zugeführt und danach in einen Ethylenoxichlorierungsprozeß eingeschleust wurde.

Der Abgasstrom (etwa 3200 Nm³/h) hatte folgende Zusammensetzung:

| | | |
|---|---|---|
| 96,20 | Mol-% | Chlorwasserstoff |
| 1,50 | Mol-% | 1,2-Dichlorethan |
| 2,23 | Mol-% | Ethylen |
| 68 | Vol.-ppm | Tetrachlorkohlenstoff |
| 8 | Vol.-ppm | Perchlorethylen |
| 6 | Vol.-ppm | Ethylchlorid |
| 2 | Vol.-ppm | 1,1-Dichlorethan |

Die vereinigten Kondensate aus den beiden Wärmetauschern (etwa 3050 kg/h) hatten nach Entspannung auf Atmosphärendruck bei einer Mischtemperatur von 10 °C folgende Zusammensetzung:

| | | |
|---|---|---|
| 96,18 | Gew.-% | 1,2-Dichlorethan |
| 3,00 | Gew.-% | Tetrachlorkohlenstoff |
| 290 | Gew.-ppm | Perchlorethylen |
| 316 | Gew.-ppm | Ethylen |
| 7 380 | Gew.-ppm | Chlorwasserstoff |
| 44 | Gew.-ppm | Ethylchlorid |
| 20 | Gew.-ppm | 1,1-Dichlorethan |

Dieses Kondensat konnte ohne zusätzliche Reinigung zusammen mit 1,2-Dichlorethan, das aus einer Ethylenoxichlorierungs-Einheit gewonnen wurde, einem Spaltofen zur Gewinnung von Vinylchlorid zugeführt werden.

Die Raumzeitleistung des Reaktors betrug 1019 kg 1,2-Dichlorethan pro m³ Katalysator und Stunde. Der Chlorumsatz lag bei nahezu 100%, die Selektivität bezüglich der Bildung von 1,2-Dichlorethan betrug 99,9%. Selbst nach einer Betriebsdauer von 2 Jahren konnten noch keinerlei Anzeichen einer Inaktivierung oder eines Kornzerfalls des Katalysators beobachtet werden.

Beispiel 2

Es wurde ein Nickelrohr als Reaktor verwendet von 350 cm Länge und 25 mm lichter Weite entsprechend einem Katalysatorvolumen von 1715 cm³. Das Nickelrohr war mit einem Mantel umgeben, indem ein auf 85 °C thermostatisiertes Öl umgepumpt wurde. Das Nickelrohr war mit einem Trägerkatalysator gefüllt. Das Trägermaterial bestand aus 5 mm großen Kugeln aus Kieselgel. Das in Strömungsrichtung ansteigende Aktivitätsprofil des Katalysators ist durch den folgenden Katalysatorfüllplan charakterisiert:

1. Drittel (Reaktor Eingang)
3 Gew.-% CuCl₂ + 3 Gew.-% MnCl₂ + 3 Gew.-% KCl
2. Drittel (Reaktor Mitte)
6 Gew.-% CuCl₂ + 6 Gew.-% MnCl₂ + 3 Gew.-% KCl
3. Drittel (Reaktor Austritt)
9 Gew.-% CuCl₂ + 9 Gew.-% MnCl₂ + 3 Gew.-% KCl

In den Reaktor wurde Chlorwasserstoff mit einem Gehalt von 12 Mol-% Chlor bei einem Druck von 1,5 bar absolut in einer Menge von 1900 Nl/h zusammen mit 255 Nl/h Ethylen eingeschleust. Das den Reaktor verlassende Reaktionsgemisch wurde bei minus 25 °C kondensiert.

Das in einer Menge von 980 g/h anfallende Kondensat hatte die folgende Zusammensetzung:

96,45 Gew.-% 1,2-Dichlorethan
0,03 Gew.-% Ethylen
3,52 Gew.-% Chlorwasserstoff

Der nicht kondensierte Gasstrom (Menge: 1699 Nl/h) enthielt:

97,49 Mol-% Chlorwasserstoff
0,83 Mol-% 1,2-Dichlorethan
1,68 Mol-% Ethylen

Die Raumzeitleistung betrug 588 g 1,2-Dichlorethan pro 1 l Katalysatorvolumen und Stunde. Die Raumströmungsgeschwindigkeit unter Normbedingungen betrug 1257 h⁻¹, die Verweilzeit des Reaktionsgemisches im Reaktor betrug 3,7 Sekunden. Der Chlorumsatz sowie die Selektivität bezüglich der Bildung 1,2-Dichlorethan betrug jeweils 100%.

Vergleichsbeispiel 1

Es wurde die Arbeitsweise des Beispiels 2 wiederholt mit den folgenden Abänderungen:

In den Reaktor wurden Chlorwasserstoff eingespeist mit einem Chloranteil von 15 Mol-%. Es wurden weiterhin die Raumströmungsgeschwindigkeiten und Verweilzeiten im Reaktor gemäß der folgenden Tabelle 1 abgeändert:

Tabelle 1

| Vers.-Nr. | Raumströmungs-geschwindigkeit $(h^{-1})$ | Verweil-zeit (sec) | Temperatur (°C) | Chlorumsatz (%) | Selektivität (%) | Raumzeit-leistung $(g/l \cdot h)$ |
|---|---|---|---|---|---|---|
| 1 | 450 | 8 | 80 | 99,4 | 99,6 | 277 |
| 2 | 670 | 1,5 | 80 | 99,2 | 99,5 | 444 |
| 3 | 670 | 4,2 | 150 | 99,5 | 99,3 | 444 |
| 4 | 670 | 4 | 200 | 99,6 | 98,9 | 444 |
| 5 | 478 | 7 | 80 | 99,5 | 99,6 | 317 |
| 6 | 209 | 16 | 80 | 99,6 | 99,7 | 139 |

Die Ergebnisse gemäß den Vergleichsversuchen 1, 5 und 6 zeigen, daß trotz erhöhter Verweilzeiten bei Raumgeschwindigkeiten unter 1000 $h^{-1}$ der Chlorumsatz nicht quantitativ gestaltet werden kann und die Raumzeitleistungen wesentlich niedriger liegen als nach dem erfindungsgemäßen Verfahren. Die Vergleichsversuche 2, 3 und 4 zeigen außerdem, daß mit zunehmender Reaktionstemperatur bei Raumströmungsgeschwindigkeiten unter 1000 $h^{-1}$ die Selektivität der Reaktionen in bezug auf Bildung von 1,2-Dichlorethan abfällt.

Für sämtliche Vergleichsversuche ist festzustellen, daß ein Chlorumsatz von lediglich 99,6% immerhin bedeutet, daß im Chlorwasserstoff nach der reaktiven Chlorumsetzung mit Ethylen noch 600 Vol.-ppm enthalten sind, die den so gewonnenen Chlorwasserstoff als Einsatzstoff für eine Ethylenoxichlorierung unbrauchbar machen.

## Patentansprüche

1. Verfahren zur Aufbereitung von bei Chlorierungsreaktionen anfallendem Chlorwasserstoff für den Ethylenoxichlorierungsprozeß durch Umsetzen von in Chlorwasserstoff enthaltenem Chlor mit Ethylen in der Gasphase in Gegenwart von Trägerkatalysatoren auf Basis Übergangsmetallchlorid, wobei Eisenchlorid ausgeschlossen ist, dadurch gekennzeichnet, daß das im Chlorwasserstoff enthaltene Chlor bei Temperaturen von 70 bis 150 °C, bei einem Druck von 1,5 bis 5 bar absolut, mit einem 5 bis 20%igen molaren Überschuß an Ethylen in Gegenwart eines Trägerkatalysators auf Basis Übergangsmetallchlorid mit in Strömungsrichtung ansteigendem Aktivitätsprofil zur Reaktion gebracht wird, wobei Raumgeschwindigkeiten von 1000 $h^{-1}$ bis 3000 $h^{-1}$ und Kontaktzeiten von 2 bis 5 s eingehalten werden und das Reaktionsprodukt nach Austritt aus dem Reaktor einer Teilkondensation unterworfen wird, wobei in zumindest einem Kondensationsschritt eine Temperatur von –20 bis –30 °C eingehalten wird.

2. Verfahren nach Anspruch 1, dadurch gekennzeichnet, daß die Reaktionstemperatur 90 bis 130 °C beträgt.

3. Verfahren nach Anspruch 1 und 2, dadurch gekennzeichnet, daß die Raumgeschwindigkeit 1200 $h^{-1}$ bis 1800 $h^{-1}$ beträgt.

4. Verfahren nach den Ansprüchen 1, 2 und 3, dadurch gekennzeichnet, daß der aufbereitende Chlorwasserstoff aus einem Perchlorierungsprozeß stammt.

## Claims

1. Process for the pretreatment of hydrogen chloride which is formed in chlorination reactions for the oxychlorination process of ethylene by reacting chlorine which is contained in hydrogen chloride with ethylene in the gas phase in the presence of supported catalysts based on transition metal chloride with the exception of iron chloride, characterized in that the chlorine contained in hydrogen chloride is reacted at temperatures between 70 and 150 °C at a pressure of 1.5 to 5 bar absolute with a 5 to 20% molar excess of ethylene in the presence of a supported catalyst based on transition metal chloride having an activity profile increasing in the flow direction, space velocities from 1,000 $h^{-1}$ to 3,000 $h^{-1}$ and contact times from 2 to 5 s being maintained and subjecting the reaction product after it leaves the reactor to a partial condensation, a temperature from –20 to –30 °C being maintained in at least one condensation step.

2. Process according to claim 1, characterized in that the reaction temperature is 90 to 130 °C.

3. Process according to claims 1 and 2, characterized in that the space velocity is 1,200 $h^{-1}$ to 1,800 $h^{-1}$.

4. Process according to claims 1, 2 and 3, characterized in that the pretreated hydrogen chloride originates from a perchlorination process.

## Revendications

1. Procédé de traitement de chlorure d'hydrogène obtenu lors de réactions de chloration, pour le procédé d'oxychloration de l'éthylène par réaction, avec l'éthylène, du chlore contenu dans le chlorure d'hydrogène, en phase gazeuse en présence de catalyseurs à base d'un chlorure de métal de transition sur un suport, à l'exclusion du chlorure de fer, procédé caractérisé en ce qu'on fait réagir le chlore contenu dans le chlorure d'hydrogène, à des températures de 70 à 150 °C, sous une pression absolue de 1,5 à 5 bars, avec un excès molaire de 5 à 20% d'éthylène en présence d'un catalyseur sur support à base d'un chlorure de métal de transition qui a un profil d'activité augmentant dans le sens de l'écoulement, en même temps qu'on maintient des vitesses spatiales de 1000 h$^{-1}$ à 3000 h$^{-1}$ et des temps de contact de 2 à 5 secondes, et l'on soumet, après sa sortie du réacteur, le produit de réaction à une condensation partielle en maintenant, dans au moins une étape de condensation, une température allant de −20 à −30 °C.

2. Procédé selon la revendication 1, caractérisé en ce que la température de réaction se situe entre 90 et 130 °C.

3. Procédé selon les revendications 1 et 2 caractérisé en ce que la vitesse spatiale se situe entre 1200 h$^{-1}$ et 1800 h$^{-1}$.

4. Procédé selon l'une quelconque des revendications 1, 2 et 3, caractérisé en ce que le chlorure d'hydrogène à traiter provient d'un procédé de perchloration.